# EUROPÄISCHE PATENTANMELDUNG

(11) **EP 2 281 499 A1**
(43) Veröffentlichungstag der Anmeldung: **09.02.2011**
(21) Anmeldenummer: 10007417.8
(22) Anmeldetag: 17.07.2010
(51) Int. Cl.: A61B 1/005, G02B 23/24

(54) **Endoskopisches Instrument**

(30) Priorität: 06.08.2009 DE 102009036424
(71) Anmelder: Richard Wolf GmbH, 75438 Knittlingen (DE)
(72) Erfinder: Wehrheim, Frank, Dipl.-Ing., 75015 Bretten (DE)
(74) Vertreter: Hemmer, Arnd

(57) **Zusammenfassung**

Die Erfindung betrifft ein endoskopisches Instrument (2) mit einem Schaft (3), welcher in zumindest einem Abschnitt (4) entlang seiner Längsachse (X) biegbar ausgebildet ist, wobei der biegbare Abschnitt (4) des Schaftes (3) eine rohrförmige Wandung aus einem elektroaktiven Polymer mit jeweils einer Mehrzahl von darin eingebetteten Steuer- (6) und Referenzelektroden (8) aufweist, welche in axialer Richtung (X) gesehen abwechselnd angeordnet und voneinander beabstandet sind, wobei sich die in axialer Richtung (X) abwechselnden Steuerelektroden (6) und Referenzelektroden (8) jeweils starr ausgebildet und in axialer Richtung (X) jeweils über am Außen- oder Innenumfang angeordnete elastische Stege (14,18) elektrisch leitend miteinander verbunden sind, sowie ein Verfahren zur Herstellung eines biegbaren Abschnittes eines solchen endoskopischen Instrumentes.

## Beschreibung

Die Erfindung betrifft ein endoskopisches Instrument mit den im Oberbegriff des Anspruchs 1 angegebenen Merkmalen.

Beispielsweise aus DE 10 2008 047 776 ist ein endoskopisches Instrument bekannt, welches einen Schaftabschnitt aus einem elektroaktiven Polymer aufweist, wobei in das elektroaktive Polymer Elektroden zur Aktivierung eingebettet sind. Diese Elektroden sind als zahnförmig ineinandergreifende Strukturen ausgebildet.

Es ist Aufgabe der vorliegenden Erfindung, ein derartiges endoskopisches Instrument derart zu verbessern, dass eine bessere Biegbarkeit bei einer einfach auszubildenden Elektrodenstruktur erreicht wird.

Diese Aufgabe wird durch ein Endoskopisches Instrument mit den im Anspruch 1 angegebenen Merkmalen gelöst. Bevorzugte Ausführungsformen ergeben sich aus den Unteransprüchen, der nachfolgenden Beschreibung sowie den beigefügten Figuren.

Das erfindungsgemäße endoskopische Instrument weist einen Schaft auf, welcher zumindest in einem Axialabschnitt, d.h. in einem Abschnitt entlang seiner Längsachse biegbar ausgebildet ist. Dieser biegbare Abschnitt weist eine rohrförmige Wandung aus einem elektroaktiven Polymer auf. In das elektroaktive Polymer ist eine Mehrzahl von Referenzelektroden eingebettet, über welche an das elektroaktive Polymer eine Spannung angelegt werden kann, um das elektroaktive Polymer zu einer Formänderung und damit zu einer Biegung des Schaftes zu veranlassen. Dazu sind die Steuer- und Referenzelektroden in axialer Richtung abwechselnd angeordnet, d. h. es ist jeweils eine Steuerelektrode zwischen zwei Referenzelektroden gelegen. Gleichzeitig sind die Elektroden beabstandet, sodass zwischen ihnen das elektroaktive Polymer gelegen ist, welches durch Anlegen einer elektronischen Spannung seine Form ändert.

Erfindungsgemäß sind die Steuer- und Referenzelektroden jeweils starr ausgebildet und jeweils in axialer Richtung über elastische, d. h. ohne bleibende Verformung biegbare, Stege elektrisch leitend miteinander verbunden. Dabei sind die Steuerelektroden untereinander über elastische Stege miteinander verbunden und die Referenzelektroden untereinander über elastische Stege elektrisch leitend miteinander verbunden. Zwischen den Steuer- und Referenzelektroden besteht keine elektrische Verbindung. Die elastischen Stege ermöglichen es, dass die starren Steuer- und Referenzelektroden sich bei Stauchung oder Ausdehnung des zwischen Ihnen gelegenen elektroaktiven Polymers in axialer Richtung relativ zueinander bewegen können. Durch die starre Ausgestaltung der Steuer- und Referenzelektroden wird eine Stabilisierung der Wandung aus dem elektroaktiven Polymer in radialer Richtung bezogen auf die Längsachse des Schaftes erreicht. Durch die elektrische Verbindung der Mehrzahl von Referenzelektroden untereinander und die elektrische Verbindung der Steuerelektroden untereinander wird darüber hinaus die Zahl der erforderlichen Anschlussleitungen reduziert, wodurch ein vereinfachter Aufbau der Elektrodenstruktur geschaffen wird. Erfindungsgemäß sind darüber hinaus die elastischen Stege entweder am Außen- oder am Innenumfang der Referenz- bzw. Steuerelektroden gelegen. D. h. die elastischen Stege sind bezogen auf die Längsachse des Schaftes radial innenliegend der Elektroden oder radial außenliegend an deren Außenseiten angeordnet. Durch diese Ausgestaltung wird eine gute Biegbarkeit des Schaftes sichergestellt. Die Referenzelektroden erstrecken sich vorzugsweise alle parallel zueinander. Entsprechend erstrecken sich auch die Steuerelektroden vorzugsweise parallel zueinander, insbesondere erstrecken sich auch die Steuer- und die Referenzelektroden parallel zueinander. So wird eine parallele Schichtung der einzelnen Elektroden in der Wandung aus elektroaktiven Polymer geschaffen.

Die Steuerelektroden und/oder die Referenzelektroden sind bevorzugt plattenförmig ausgebildet und erstrecken sich quer zur Längsachse des Schafts. Die Oberflächen der plattenförmigen Elektroden erstrecken sich somit in Durchmesserebenen bezogen auf die Längsachse des Schaftes. Die plattenförmige Struktur ergibt sich dadurch, dass die Elektroden in radialer Richtung eine größere Ausdehnung haben als in axialer Richtung, d. h. als in Richtung parallel zur Längsachse. In Umfangsrichtung haben die Elektronen bevorzugt ohnehin eine größere Ausdehnung, d. h. sie erstrecken sich über einen großen Umfangsbereich ggf. über den vollständigen Umfang der Wandung. Durch die plattenförmige Ausgestaltung wird eine große aktive Elektrodenfläche geschaffen und gleichzeitig wird aufgrund der größeren Elektrodendicke in radialer Richtung in radialer Richtung eine größere Stabilität der Wandung erzielt.

Ferner ist der biegbare Abschnitt zweckmäßigerweise in Umfangsrichtung gesehen zumindest in zwei getrennt aktivierbare Aktorfelder aufgeteilt, in welchen jeweils in axialer Richtung gesehen die zuvor beschriebenen Steuer- und Referenzelektroden einander abwechselnd und beabstandet angeordnet sind. Weiter bevorzugt sind drei oder mehr Aktorfelder vorgesehen, welche in Umfangsrichtung vorzugsweise gleichmäßig verteilt in der Wandung angeordnet sind. Dadurch, dass die Aktorfelder separat aktiviert, d. h. deren Elektroden separat mit Spannung beaufschlagt werden können, ist es dann möglich den Schaft in beliebige Richtungen zu biegen, je nachdem welches oder welche der Aktorfelder aktiviert wird/werden. In den Aktorfeldern sind die Elektroden so, wie oben beschrieben, in abwechselnder Anordnung der Steuer- und Referenzelektroden mit den elektrischen Verbindungen durch elastische Stege angeordnet. Die einzelnen Aktorfelder sind jeweils bevorzugt so ausgestaltet, dass sich die Elektroden der einzelnen Aktorfelder gemeinsam vorzugsweise im Wesentlichen über den gesamten Umfang der Wandung erstrecken. Auf diese Weise wird eine größtmögliche Stabilisierung der Wandung durch die Elektroden in radialer Richtung erreicht.

Die Referenzelektroden der mehreren Aktorfelder sind vorzugsweise elektrisch leitend miteinander verbunden. Auf diese Weise kann die Zahl der Anschlussleitungen reduziert werden, da so die Referenzelektroden mehrerer, vorzugsweise aller Aktorfelder über eine gemeinsame Anschlussleitung elektrisch angeschlossen werden können.

Dazu sind weiter bevorzugt die jeweils in derselben Durchmesserebene bezogen auf die Längsachse des Schaftes gelegenen Referenzelektroden der mehreren Aktorfelder in Umfangsrichtung elektrisch leitend miteinander verbunden. Die Verbindung der in unterschiedlichen Durchmesserebenen gelegenen Referenzelektroden in axialer Richtung erfolgt über die oben beschriebenen elastischen Stege.

Weiter bevorzugt sind die jeweils in einer Durchmesserebene gelegenen Referenzelektroden der mehreren Aktorfelder in Umfangsrichtung auch mechanisch miteinander verbunden. Die Verbindung stellt zweckmäßigerweise sowohl die mechanische als auch die elektrische Verbindung her. Auf diese Weise wird durch die elektrische Verbindung in Umfangsrichtung gleichzeitig eine mechanische Stabilisierung der Schaftwandung erreicht, da die Referenzelektroden so Ringanordnungen bilden. Gegenüber den eigentlichen Elektrodenflächen der Referenzelektroden sind die Verbindungen zwischen den einzelnen Aktorfeldern jedoch ggf. in radialer Richtung ausgedünnt gefertigt, sodass eine gewisse Biegbarkeit in Umfangsrichtung erreicht wird, welche, wie weiter unten beschrieben wird, insbesondere für die Herstellung von Vorteil sein kann.

Weiter bevorzugt haben die Referenzelektroden der mehreren Aktorfelder in axialer Richtung eine gemeinsame elektrische Verbindung. D. h. wenn die Referenzelektroden, welche in derselben Durchmesserebene gelegen sind, in Umfangsrichtung elektrisch leitend miteinander verbunden sind, ist es ausreichend zwei in axialer Richtung benachbarte Referenzelektroden, zwischen denen eine Steuerelektrode gelegen ist, in axialer Richtung nur in einem der Aktorfelder durch einen elastischen Steg miteinander zu verbinden. In den anderen Aktorfeldern sind dann derartige elastische Stege zur Verbindung dieser zwei Referenzelektroden bzw. Referenzelektroden-Ringe nicht erforderlich. Auf diese Weise wird die Zahl der axialen Verbindungen zwischen den Referenzelektroden reduziert. So wird zum einen die Biegbarkeit des Instrumentes erhöht und zum anderen die Fertigung der Elektrodenstruktur vereinfacht.

Ferner ist es bevorzugt, dass zwei in axialer Richtung benachbarte Referenzelektroden über einen elastischen Steg miteinander verbunden sind, welcher in Umfangsrichtung gesehen mit dem ersten Ende einer ersten Referenzelektrode und einem entgegengesetzten zweiten Ende der zweiten benachbarten Referenzelektrode verbunden ist. Der Steg kreuzt dabei in radialer Projektion gesehen die zwischen den Referenzelektroden gelegene Steuerelektrode, erstreckt sich jedoch seitlich am Innen- oder am Außenumfang der Steuerelektrode vorbei, ohne diese zu kontaktieren. Der elastische Steg bildet mit den verbundenen Referenzelektroden eine Z-förmige Struktur. Durch diese Struktur wird die Elastizität des Steges und die axiale Beweglichkeit der beiden Referenzelektroden zueinander gewährleistet.

Die gemeinsamen axialen Verbindungen der einzelnen Referenzelektroden sind weiter bevorzugt abwechselnd verteilt in mehreren Aktorfeldern angeordnet. D. h. bei der Vielzahl von in axialer Richtung übereinanderliegenden Referenzelektroden sind die axialen Verbindungen zwischen den einzelnen Referenzelektroden nicht alle in einem einzigen Aktorfeld angeordnet, sondern über mehrere, vorzugsweise alle Aktorfelder verteilt. So kann beispielsweise ein insgesamt schraubenförmiger Verlauf der axialen Verbindung zwischen den Referenzelektroden über den Umfang der Wandung ausgebildet sein. Auf diese Weise wird eine gleichmäßige Biegbarkeit des Schaftes in alle radialen Richtungen gewährleistet. Es gibt keine Biegerichtung, welche durch die Anordnung der elektrischen Verbindung zwischen den Referenzelektroden stärker beeinträchtigt wäre als andere.

Besonders bevorzugt ist die axiale Verbindung der Referenzelektroden somit als ein schraubenförmig über die Wandung bzw. in diese eingebettet verlaufender Steg ausgebildet. Dieser Steg verläuft entweder am Innenumfang der Referenzelektroden oder am Außenumfang der Referenzelektroden und verbindet alle Referenzelektroden-Ringe in axialer Richtung miteinander.

Weiter bevorzugt sind auch jeweils zwei benachbarte Steuerelektroden eines einzelnen Aktorfeldes über einen elastischen Steg miteinander verbunden, welcher in Umfangsrichtung gesehen mit dem ersten Ende einer ersten Steuerelektrode und einem entgegengesetzten zweiten Ende der zweiten benachbarten Steuerelektrode verbunden ist. Zwischen den zwei benachbarten Steuerelektroden ist dann jeweils eine Referenzelektrode gelegen, wobei der die Steuerelektroden verbindende Steg die Referenzelektroden in der radialen Projektion gesehen kreuzt, aber am Außen- oder Innenumfang die Referenzelektrode seitlich passiert. Durch die so beschriebene Verbindung zwischen zwei Steuerelektroden ist ebenfalls eine Z-förmige Struktur bestehend aus zwei Steuerelektroden und dem dazwischenliegenden Steg geschaffen. Wenn alle in axialer Richtung übereinander liegenden Steuerelektroden eines Aktorfeldes auf diese Weise verbunden sind, wird somit insgesamt eine zickzackförmige Elektrodenstruktur geschaffen, wobei die bei vertikaler Ausrichtung der Längsachse horizontal gelegenen Schenkel der zickzackförmigen Struktur von den Steuerelektroden und die schräg verlaufenden Schenkel durch die elastischen Stege gebildet werden. Auf diese Weise wird ebenfalls eine gute Elastizität und Verformbarkeit der elektrischen Verbindungsstege erreicht, sodass bei Verformung des elektroaktiven Polymers sich die Steuerelektroden aufeinander zu bzw. voneinander weg bewegen können.

Besonders bevorzugt sind die elastischen Stege welche die Referenzelektroden miteinander verbinden und/oder die elastischen Stege, welche die Steuerelektroden miteinander verbinden an der radial innengelegenen Umfangsseite der Steuer- und Referenzelektroden angeordnet. Weiter bevorzugt sind sowohl die Stege, welche die Steuerelektroden miteinander verbinden, als auch die Stege, welche die Referenzelektroden miteinander verbinden, an der radial innengelegenen, d.h. inneren Umfangsseite der Steuer- und Referenzelektroden bzw. der Wandung gelegen. Auf diese Weise sind die Elektroden im Inneren geschützt und die Verformungswege bei Biegung, welche die elastischen Stege vollführen müssen, sind minimiert.

Die Erfindung betrifft darüber hinaus ein Verfahren zur Herstellung eines endoskopischen Instruments gemäß der vorangehenden Beschreibung. Gemäß diesem Verfahren wird der biegbar ausgebildete Abschnitt des Schaftes in der Weise gefertigt, dass zunächst die Anordnung der Steuer- und Referenzelektroden sowie die diese verbindenden elastischen Stege als Struktur in einer planen Ebene ausgebildet wird. Dabei wechseln sich die Steuer- und Referenzelektroden in Erstreckungsrichtung der Ebene ab, d. h. in dem Fall, dass die Elektroden, wie oben beschrieben plattenförmig ausgebildet sind, erstrecken sich die Oberflächen der Platten normal zu der Ebene, in welcher die Struktur der Steuer- und Referenzelektroden ausgebildet wird. Anschließend wird die Struktur in ein elektroaktives Polymer eingegossen, sodass eine plane Platte aus elektroaktivem Polymer mit eingebetteten Elektroden geschaffen wird. Im nächsten Schritt wird diese Struktur bestehend aus elektroaktivem Polymer und eingegossenen Elektroden dann rohrförmig zu der rohrförmigen Wandung gebogen. Auf diese Weise lässt sich die rohrförmige Gestaltung mit den eingebetteten Elektroden auf relativ einfache Weise herstellen.

Bevorzugt wird dann nach dem Biegen die rohrförmige Wandung an Ihrer Außenseite mit einem elastischen Polymer vergossen. Diese hält die in der zuvor beschriebenen Weise gebogene Wandung dann außen zusammen.

Um die in der Ebene ausgebildete Elektrodenstruktur ring- bzw. rohrförmig biegen zu können, ist es bevorzugt, dass die oben beschriebenen elektrischen Verbindungen zwischen den Referenzelektroden der einzelnen Aktorfelder, welche in Umfangsrichtung verlaufen, in radialer Richtung dünner als die Referenzelektroden ausgebildet sind, sodass in diesen Bereichen eine Biegung erfolgt und die Referenz- und Steuerelektroden selber nicht gebogen werden müssen.

Nachfolgend wird die Erfindung beispielhaft anhand der beigefügten Figuren beschrieben. In diesen zeigt:
- Fig. 1: ein endoskopisches Instrument gemäß der Erfindung,
- Fig. 2: schematisch die Anordnung von Elektroden in einem biegbaren Abschnitt des Instrumentes gemäß Fig. 1,
- Fig. 3: beispielhaft die Anordnung von Steuer- und Referenzelekt- roden,
- Fig. 4: einen vergrößerten Ausschnitt aus Fig. 3,
- Fig. 5a - 5c: schematisch die Verformung des Schaftes und
- Fig. 6: die Gesamtanordnung der Elektroden in dem Schaft.

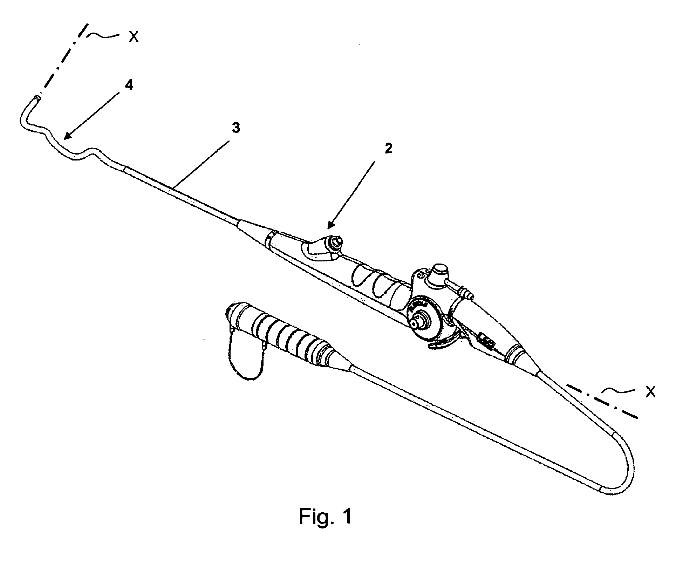

Fig. 1 zeigt ein Beispiel für ein endoskopisches Instrument gemäß der Erfindung. Es handelt sich dabei um ein Endoskop 2 mit einem Schaft 3 dessen distaler Abschnitt 4 flexibel bzw. biegbar ausgebildet ist. Erfindungsgemäß wird der distale Abschnitt 4 aus einer rohrförmigen Wandung aus einem elektroaktiven Polymer gebildet, in welches Steuerelektroden 6 und Referenzelektroden 8 eingebettet sind. Über die Steuerelektroden 6 und Referenzelektroden 8 kann das elektroaktive Polymer mit einer elektrischen Spannung beaufschlagt werden, wodurch eine Verformung des elektroaktiven Polymers erreicht wird. Diese Verformung wird zur Auslenkung bzw. Biegung des distalen Abschnittes 4 des Endoskops 2 genutzt.

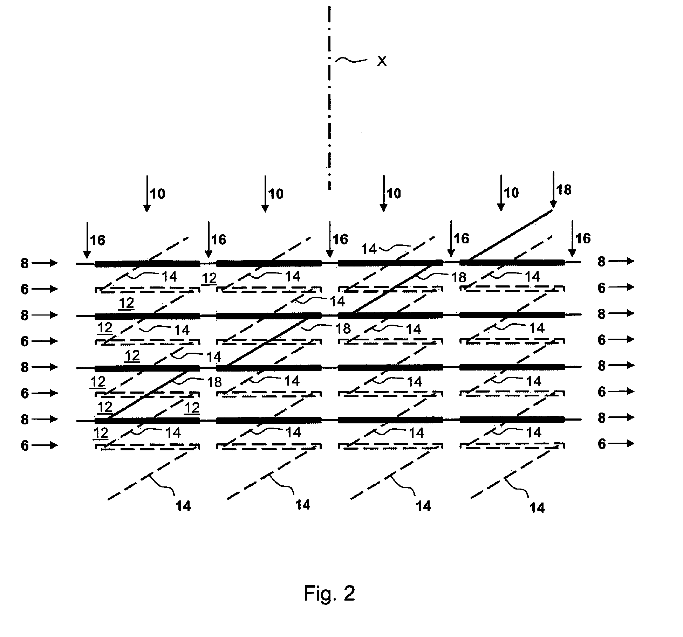

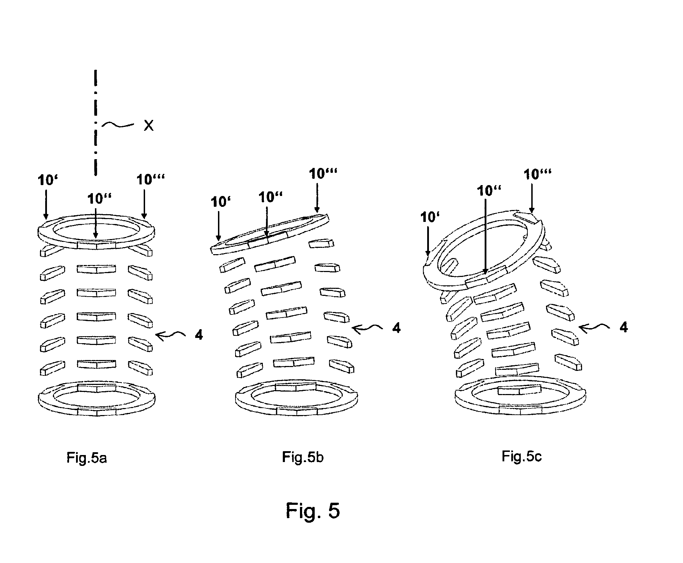

Fig. 2 zeigt schematisch die erfindungsgemäße Anordnung von Referenzelektroden 8 und Steuerelektroden 6. Verteilt um die Längsachse X des Schaftes 3 sind in der Schaftwandung des verform- bzw. biegbaren Abschnittes 4 mehrere Aktorfelder 10 angeordnet, welche kontrahierbare Sehnen bilden. In den einzelnen Aktorfeldern sind jeweils in axialer Richtung abwechselnd übereinander die Steuerelektroden 6 und die Referenzelektroden 8 angeordnet, wobei die Referenzelektroden 8 und Steuerelektroden 6 jeweils voneinander beabstandet sind, sodass in den so zwischen den Elektroden gebildeten Zwischenräumen 12 das elektroaktive Polymer angeordnet werden kann. Dazu werden die Elektroden 6 und 8 in das elektroaktive Polymer eingebettet. Durch das Anlegen einer Spannung zwischen den Steuerelektroden 6 und den Referenzelektroden 8 wird das elektroaktive Polymer in den Zwischenräumen 12 kontrahiert, sodass das gesamte Aktorfeld 10 bzw. die gesamte so gebildete Sehne 10 kontrahiert wird und der biegbare Abschnitt 4 in die entsprechende Richtung ausgelenkt wird, wie anhand der Fig. 5a bis 5c gezeigt ist. Dort ist ein Beispiel mit drei Sehnen schematisch dargestellt. In Fig. 5a ist keine der Sehnen 10', 10" und 10"' kontrahiert, d. h. keine Spannung an die entsprechenden Steuer- 6 und Referenzelektroden 8 angelegt. In der Darstellung gemäß Fig. 5b ist die Sehne 10' durch Anlegen einer Spannung an deren Elektroden kontrahiert, sodass der Schaft zur Sehne 10' hin ausgelenkt wird. Im Beispiel gemäß Fig. 5c sind die Sehnen 10' und 10" durch Anlegen von Spannung an deren Elektroden kontrahiert, sodass der Schaft bzw. dessen biegbarer Abschnitt 4 in eine Winkelrichtung zwischen den Sehnen 10' und 10" ausgelenkt wird.

Erfindungsgemäß sind, wie in Fig. 2 gezeigt, die Steuerelektroden 6, jedes der Aktorfelder 10 jeweils über elastische Stege 14 elektrisch leitend miteinander verbunden. Die elastischen Stege 14 erstrecken sich schräg zwischen zwei zueinander benachbarten und zueinander parallelen Steuerelektroden 6, sodass in radialer Projektion auf die Längsachse X gesehen zwei benachbarte Steuerelektroden 6 mit dem dazwischenliegenden Steg 14 eine Z-förmige Struktur bilden. Die Vielzahl übereinander geschichteter Steuerelektroden 6 mit den dazwischenliegenden elastischen Stegen 14 bildet so eine zickzackförmige Struktur. Durch diese elektrische Verbindung aller Steuerelektroden 6 eines Aktorfeldes 10 wird erreicht, dass durch eine einzige Anschlussleitung sämtliche Steuerelektroden 6 gleichzeitig mit Spannung beaufschlagt werden können. Dadurch das die Steuerelektroden 6 der einzelnen Aktorfelder 10 jeweils voneinander getrennt sind, ist es möglich die Steuerelektroden 6 der einzelnen Aktorfeldler 10 separat mit Spannung zu beaufschlagen und somit jedes Aktorfeld 10 separat zu aktivieren.

Im gezeigten Beispiel sind lediglich mehrere Aktorfelder 10 umfänglich um die Längsachse X in der Wandung des biegbaren Abschnittes 4 angeordnet. Es ist jedoch zu verstehen, dass auch in axialer Richtung X mehrere getrennt aktivierbare Aktorfelder 10 hintereinander angeordnet werden können, um eine schlangenlinienförmige Biegung des distalen Abschnittes 4 zu erreichen, wie sie in Fig. 1 gezeigt ist.

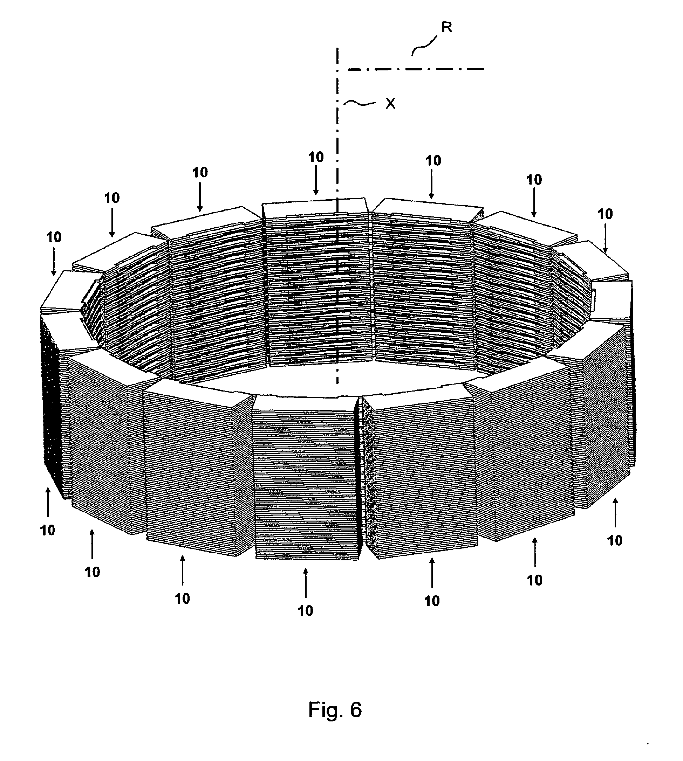

Wie darüber hinaus in Fig. 2 zu erkennen ist, sind die Referenzelektroden 8, welche jeweils in derselben Durchmesserebene bezogen auf die Längsachse X liegen, über Stege 16 in Umfangsrichtung miteinander verbunden, sodass die Referenzelektroden 8 mehrerer, vorzugsweise aller Aktorfelder 10 miteinander verbunden sind. So werden übereinanderliegende ringförmige Strukturen von Referenzelektroden 8 mit dazwischenliegenden Stegen 16 geschaffen. Die Stege 16 sind gegenüber den Referenzelektroden 8 flacher ausgebildet, sodass in diesem Bereich eine Biegung möglich ist, um die Elektrodenanordnung in die in Fig. 6 gezeigte rohförmige Form zu biegen.

Um die in axialer Richtung übereinanderliegenden Referenzelektroden 8 bzw. gebildeten Ringe aus Referenzelektroden 8 elektrisch leitend miteinander zu verbinden, sind elastische Stege 18 vorgesehen. Die elastischen Stege 18 erstrecken sich wie die elastischen Stege 14 schräg zwischen zwei benachbarte Referenzelektroden 8, sodass eine erste Referenzelektrode 8 an einem ersten Umfangsende von dem Steg 18 kontaktiert wird und die zweite benachbarte Referenzelektrode 8 am entgegengesetzten Umfangsende von dem elastischen Steg 18 kontaktiert wird. Da alle Referenzelektroden 8 über mehrere Aktorfelder 10 miteinander verbunden sind, ist es nicht erforderlich, in jedem der Aktorfelder 10 alle Referenzelektroden 8 über elastische Stege 18 miteinander zu verbinden. Vielmehr sind zwei zueinander benachbarte ringförmige Anordnungen von Referenzelektroden 8 nur in einem der Aktorfelder 10 über einen elastischen Steg 18 miteinander verbunden. Gleichzeitig sind diese elastischen Stege 18 so über die einzelnen Aktorfelder 10 verteilt, dass sie immer um ein Aktorfeld 10 versetzt sind, sodass insgesamt von den elastischen Stegen 18 ein im Wesentlichen schraubenförmiger Verlauf um die Elektrodenanordnung gebildet wird.

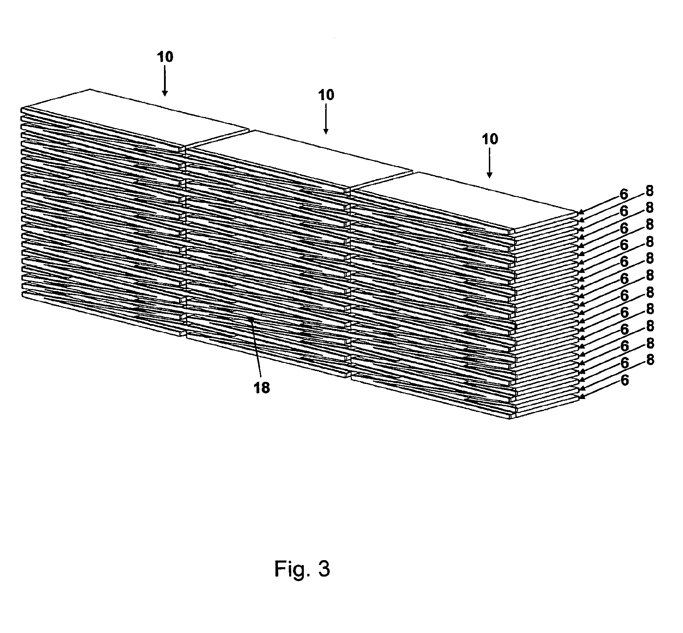

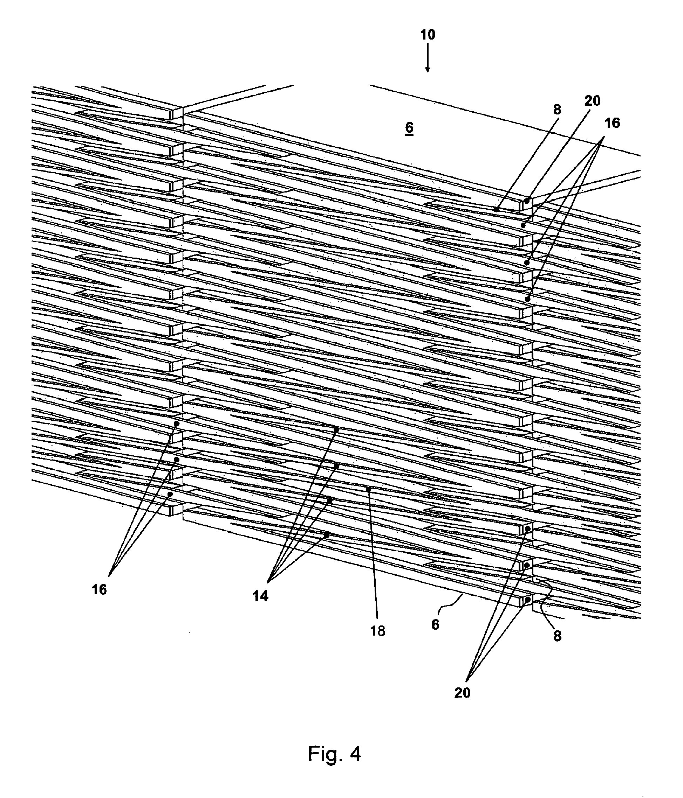

Die Steuerelektroden 6 und die Referenzelektroden 8 sind, wie in Fig. 3 und Fig. 4 sowie Fig. 6 zu erkennen ist, jeweils plattenförmig ausgebildet, d. h. sie weisen in radialer Richtung R bezogen auf die Längsachse X eine größere Ausdehnung auf als in Richtung der Längsachse X. Auf diese Weise wird eine starre Ausgestaltung der Elektroden 6 und 8 insbesondere in radialer Richtung erreicht. Damit bewirken die Referenzelektroden 8 und Steuerelektroden 6 gleichzeitig eine Verstärkung der Wandung aus elektroaktivem Polymer in radialer Richtung. Die elastischen Stege 14 und 18 ermöglichen jedoch eine Bewegung der Referenzelektroden 8 und Steuerelektroden 6 in axialer Richtung X aufeinander zu, wenn das zwischenliegende elektroaktive Polymer in dieser Richtung verformt wird. Dadurch wird die Biegbarkeit des Schaftes realisiert. Die Fig. 3 und 4 zeigen wie die Struktur bestehend aus Steuerelektroden 6 und Referenzelektroden 8 sowie den Stegen 14, 16 und 18 realisiert wird. Wie in Fig. 3 und 4 gezeigt, wird die Elektrodenstruktur vorzugsweise zunächst als plane, d. h. nicht zu einem Rohr gebogene Struktur ausbildet. Die so ausgebildete Elektrodenstruktur kann dann in das elektroaktive Polymer eingegossen und gemeinsam mit diesem in die in Fig. 6 gezeigte rohrförmige Form gebogen werden (in Fig. 6 ist das elektroaktive Polymer nicht dargestellt). Anschließend kann diese Struktur dann außen noch einmal mit einem elastischen Polymer umgossen werden, um die so gebildete Form zu fixieren.

Wie in Fig. 6 zu erkennen ist, kann eine Vielzahl von Aktorfeldern 10 gleichmäßig um den Umfang der Längsachse X verteilt angeordnet werden, sodass der Schaft sehr genau steuerbar in unterschiedliche radiale Richtungen auslenkbar sind. Gleichzeitig grenzen die Referenzelektroden 8 und Steuerelektroden 6 der einzelnen Aktorfelder 10 fast direkt aneinander an, sodass in allen Umfangsbereichen Elektroden angeordnet sind und so die Wandung in allen Umfangsbereichen in radialer Richtung stabilisiert ist. Wie in Fig. 6 zu erkennen ist, ist die Elektrodenanordnung gemäß Fig. 3 so gebogen worden, dass die elastischen Stege 14 und 18 am Innenumfang der Elektrodenanordnung gelegen ist. So werden die Verformungswege für die elastischen Stege bei der Verbiegung kurz gehalten.

In Fig. 3 und 4 ist zu erkennen, wie die Verbindungsstege an den plattenförmigen Steuerelektroden 6 und Referenzelektroden 8 befestigt sind. An der Stirnkanten, welche später dem Innenumfang bezogen auf die Längsachse X zugewandt sind, sind die Stege über elektrisch leitende Abstandshalter 20 angebracht. Die Abstandshalter 20 bewirken, dass die elastischen Stege 14 die zwischenliegenden Referenzelektroden 8 seitlich passieren können, ohne diese zu berühren. Entsprechend wird sichergestellt, dass die elastischen Stege 18 die zwischenliegenden Steuerelektroden 6 seitlich passieren können, ohne die Elektroden zu berühren.

### Bezugszeichenliste

- 2 -: Endoskop
- 3 -: Schaft
- 4 -: Distaler Abschnitt
- 6 -: Steuerelektroden
- 8 -: Referenzelektroden
- 10 -: Aktorfelder bzw. Sehnen
- 12 -: Zwischenräume
- 14 -: Elastische Stege
- 16 -: Stege
- 18 -: Elastische Stege
- 20 -: Abstandshalter
- X -: Instrumentenlängsachse
- R -: Radiale Richtung

## Patentansprüche

1. Endoskopisches Instrument (2) mit einem Schaft (3), welcher in zumindest einem Abschnitt (4) entlang seiner Längsachse (X) biegbar ausgebildet ist, wobei der biegbare Abschnitt (4) des Schaftes (3) eine rohrförmige Wandung aus einem elektroaktiven Polymer mit jeweils einer Mehrzahl von darin eingebetteten Steuer- (6) und Referenzelektroden (8) aufweist, welche in axialer Richtung (X) gesehen abwechselnd angeordnet und voneinander beabstandet sind,
**dadurch gekennzeichnet, dass**
sich die in axialer Richtung (X) abwechselnden Steuerelektroden (6) und Referenzelektroden (8) jeweils starr ausgebildet und in axialer Richtung (X) jeweils über am Außen- oder Innenumfang angeordnete elastische Stege (14, 18) elektrisch leitend miteinander verbunden sind.

2. Endoskopisches Instrument nach Anspruch 1, **dadurch gekennzeichnet, dass** die Steuerelektroden (6) und/oder die Referenzelektroden (8) plattenförmig ausgebildet sind und sich quer zur Längsachse (X) erstrecken.

3. Endoskopisches Instrument nach Anspruch 1 oder 2, **dadurch gekennzeichnet, dass** der biegbare Abschnitt (4) des Schaftes (3) in Umfangsrichtung gesehen zumindest zwei getrennt aktivierbare Aktorfelder (10) aufweist, in welchen jeweils in axialer Richtung (X) gesehen einander abwechselnd Steuer- (6) und Referenzelektroden (8) angeordnet sind.

4. Endoskopisches Instrument nach Anspruch 3, **dadurch gekennzeichnet, dass** die Referenzelektroden (8) der mehreren Aktorfelder (10) elektrisch leitend miteinander verbunden sind.

5. Endoskopisches Instrument nach Anspruch 4, **dadurch gekennzeichnet, dass** die jeweils in einer Durchmesserebene gelegenen Referenzelektroden (8) der mehreren Aktorfelder (10) in Umfangsrichtung elektrisch leitend miteinander verbunden sind.

6. Endoskopisches Instrument nach Anspruch 4, **dadurch gekennzeichnet, dass** die jeweils in einer Durchmesserebene gelegenen Referenzelektroden (8) der mehreren Aktorfelder (10) in Umfangsrichtung mechanisch miteinander verbunden sind.

7. Endoskopisches Instrument nach Anspruch 4 oder 5, **dadurch gekennzeichnet, dass** die Referenzelektroden (8) der mehreren Aktorfelder (10) in axialer Richtung (X) eine gemeinsame elektrische Verbindung (18) aufweisen.

8. Endoskopisches Instrument nach Anspruch 7, **dadurch gekennzeichnet, dass** zwei in axialer Richtung (X) benachbarte Referenzelektroden (8) über einen elastischen Steg (18) miteinander verbunden sind, welcher in Umfangsrichtung gesehen mit dem ersten Ende einer ersten Referenzelektrode (8) und einem entgegengesetzten zweiten Ende der zweiten benachbarten Referenzelektrode (8) verbunden ist.

9. Endoskopisches Instrument nach Anspruch 7 oder 8, **dadurch gekennzeichnet, dass** die gemeinsamen axialen Verbindungen (18) der einzelnen Referenzelektroden (8) abwechselnd verteilt in den mehreren Aktorfeldern (10) angeordnet sind.

10. Endoskopisches Instrument nach Anspruch 9, **dadurch gekennzeichnet, dass** die axiale Verbindung der Referenzelektroden (8) als ein schraubenförmig in die Wandung eingebettet verlaufender Steg (18) ausgebildet ist.

11. Endoskopisches Instrument nach einem der Ansprüche 3 bis 10, **dadurch gekennzeichnet, dass** jeweils zwei benachbarte Steuerelektroden (6) eines einzelnen Aktorfeldes (10) über einen elastischen Steg (14) miteinander verbunden sind, welcher in Umfangsrichtung gesehen mit dem ersten Ende einer ersten Steuerelektrode (6) und einem entgegengesetzten zweiten Ende der zweiten benachbarten Steuerelektrode (6) verbunden ist.

12. Endoskopisches Instrument nach einem der vorangehenden Ansprüche, **dadurch gekennzeichnet, dass** die elastischen Stege (18), welche die Referenzelektroden (8) miteinander verbinden, und/oder die elastischen Stege (14), welche die Steuerelektroden (6) miteinander verbinden an der radial innen gelegenen Umfangsseite der Steuer- (6) und Referenzelektroden (8) angeordnet sind.

13. Verfahren zur Herstellung eines endoskopischen Instrumentes nach einem der vorangehenden Ansprüche, bei welchem die Anordnung der Steuer- (6) und Referenzelektroden (8) sowie der diese verbindenden elastischen Stege (14, 18) zunächst als Struktur in einer planen Ebene ausgebildet wird, wobei sich Steuer-(6) und Referenzelektroden (8) in Erstreckungsrichtung der Ebene abwechseln,
die Struktur anschließend in ein elektroaktives Polymer eingegossen und dann
zu einer rohrförmigen Wandung gebogen wird.

14. Verfahren nach Anspruch 13, bei welchem nach dem Biegen die rohrförmige Wandung an ihrer Außenseite mit einem elastischen Polymer vergossen wird.
